# EUROPEAN PATENT APPLICATION

(11) **EP 1 905 343 A1**
(43) Date of publication of application: **02.04.2008**
(21) Application number: 06732436.8
(22) Date of filing: 27.04.2006
(51) Int. Cl.: A61B 1/00, A61B 1/12, A61B 19/02, A61G 12/00, A61L 2/10

(54) **MEDICAL INSTRUMENT STORAGE DEVICE**

(30) Priority: 19.07.2005 JP 2005209207; 19.07.2005 JP 2005209208
(71) Applicant: OLYMPUS MEDICAL SYSTEMS CORP., Shibuya-ku Tokyo 151-0072 (JP)
(72) Inventor: NOGUCHI, Toshiaki, Olympus Medical Systems Corp., Shibuya-ku, Tokyo 151-0072 (JP); SUZUKI, Eiri, Olympus Medical Systems Corp., Shibuya-ku, Tokyo 151-0072 (JP)
(74) Representative: von Hellfeld, Axel
(86) International application number: PCT/JP2006/308885
(87) International publication number: WO 2007/010657

(57) **Abstract**

A medical instrument storage system includes a power feeder connected to a power source (4), a main body (3) which includes a power receiver (14) for receiving a contactless power supply from the power feeder, and a holding portion (2) for holding a medical instrument, a sanitation keeping unit (13, 35) connected to the power receiver and driven by the power supplied from the power feeder through the power receiver to keep the medical instrument held in the holding portion in a sanitary state, a moving unit (12) for making the main body movable, an electric storage unit (91) provided in the holding vessel for storing power supplied from the power feeder, and a light emitting unit (35) connected to the electric storage unit provided in the holding vessel for emitting a light ray with a predetermined wavelength toward the medical instrument.

## Description

### Technical Field

The present invention relates to a medical instrument storage system, and more particularly, to a medical instrument storage system for storing and carrying a medical instrument, which is structured to allow easy power supply required for keeping the stored instrument in good sanitary condition and easy sanitary control and information management of the stored medical instrument through the power supply.

### Background Art

Generally, the used medical instrument, for example, the endoscope that has been used for the endoscopic inspection will be subjected to the washing/sterilizing process in accordance with the predetermined procedure, and then stored under the predetermined environment.

Specifically, the used medical instrument, for example, the endoscope used for the inspection is carried to the sink for prewashing the outer surface, and then further carried to the washer/sterilizer for the full washing.

The thus washed/sterilized medical instrument, for example, the endoscope is stored inside the storage unit for the medical instrument structured to maintain the sanitary environment until the use for the next inspection. The medical instrument, especially the endoscope is normally stored in the special storage unit.

In the case where the used medical instrument such as the used endoscope is subjected to the washing/sterilizing operation after the endoscopic inspection, the operator is expected to carry the subject medical instrument with his/herhands from place to place, for example, from the inspection room to the sink for prewashing, from the sink to the washer/sterilizer for the full washing, and from the washer/sterilizer to the storage unit after the washing/sterilizing operation. The operator is further expected to take the subject medical instrument by hand from the storage unit to be carried to the inspection room for the use in the next inspection.

It is necessary to carry the cleaned medical instrument to the storage unit for storage, and to carry the medical instrument stored in the storage unit to the place for the inspection with care especially in view of sanitation.

The generally employed storage unit for the medical instrument is provided with a mercury lamp which emits the UV ray for sterilization. The mercury lamp for sterilization is structured to keep the medical instrument stored in the storage unit in good sanitary condition through UV emission.

The aforementioned storage unit requires predetermined power from the general commercial power source, for example, to allow emission of the mercury lamp for sterilization.

A power cable extends from the generally employed storage unit for the medical instrument, and a plug of the power cable is connected to the outlet on the wall surface of the room where the storage unit is placed such that the power is supplied to the storage unit.

The storage unit for the endoscope as disclosed in Japanese Unexamined Patent Application Publication No. 10-262902 is structured to be installed on the side surface of a body of the cart on which the light source device and the processors are mounted. The aforementioned storage unit is structured to store the endoscope which is hung therein. The storage unit is also provided with the sterilizing lamp therein for keeping the inside in good sanitary condition.

The generally employed storage unit as disclosed in Japanese Unexamined Patent Application Publication No. 10-262902 requires the power source to allow emission of the mercury lamp and the power cable for power supply. When moving the body of the cart including the storage unit, the power cable has to be detached.

Meanwhile, it is preferable to keep the user from contacting the equipment other than the medical instrument for the purpose of maintaining the sanitary condition of the medical instrument. As the power cable is likely to be pulled on the floor surface, the user is required to change the gloves for handling the power cable.

The movement of the generally employed storage unit for the medical instrument has been regarded as the work which accompanies the extreme difficulty in the control of the sanitary condition of the medical instrument stored in the storage unit.

The medical instrument stored in the generally employed storage unit for the medical instrument is required to be placed in the other vessel so as to be moved owing to the sterilizing lamp inside the storage unit. In the aforementioned case, the other vessel for the carriage is formed as the cabinet having the sterilizing lamp so as to be moved for maintaining the sanitary condition for an elongated time.

The cabinet which contains the sterilizing lamp is required to be connected to the electrical outlet for power supply to the sterilizing lamp, thus interfering in easy movement.

In view of the aforementioned points, it is an object of the present invention to provide a medical instrument storage system capable of maintaining the sanitary condition of the medical instrument for an elongated time, and easily performing the sanitary control while allowing easy movement of the medical instrument.

### Disclosure of Invention

### Means for Solving the Problem

For the purpose of achieving the aforementioned object, a first medical instrument storage system according to the present invention includes a power feeder connected to a power source, a main body which includes a power receiver for receiving a contactless power supply from the power feeder, and a holding portion for holding a medical instrument, sanitation keeping means (unit) connected to the power receiver and driven by the power supplied from the power feeder through the power receiver to keep the medical instrument held in the holding portion in a sanitary state, and moving means (unit) for making the main body movable.

The aforementioned first medical instrument storage system is allowed to easily control the sanitary condition of the medical instrument appropriately as well as to move the medical instrument easily.

A second medical instrument storage system according to the present invention includes a power feeder connected to a power source, a main body detachably provided with a holding vessel including a holding portion for holding a medical instrument, an electric storage unit provided in the holding vessel for storing power supplied from the power feeder, and a light emitting unit connected to the electric storage unit provided in the holding vessel for emitting a light ray with a predetermined wavelength toward the medical instrument.

The aforementioned second medical instrument storage system according to the present invention is allowed to keep the medical instrument in the sanitary condition for an elongated time, to move the medical instrument easily while being kept in the sanitary condition, and to perform the appropriate sanitary control.

A third medical instrument storage system according to the present invention includes a power feeder connected to a power source, a main body which includes a power receiver for receiving a contactless power supply from the power feeder, and a holding portion for holding a medical instrument, a sanitation keeping unit connected to the power receiver and driven by the power supplied from the power feeder through the power receiver to keep the medical instrument held in the holding portion in a sanitary state, a moving unit for making the main body movable, an electric storage unit provided in the holding vessel for storing power supplied from the power feeder, and a light emitting unit connected to the electric storage unit provided in the holding vessel for emitting a light ray with a predetermined wavelength toward the medical instrument.

### Brief Description of the Drawings

Fig. 1 is an external perspective view showing the entire configuration of a medical instrument storage system (trolley system) of a first embodiment according to the present invention.
Fig. 2 is a block diagram primarily showing the electrical configuration in the medical instrument storage system (trolley system) shown in Fig. 1.
Fig. 3 is an explanatory view showing the function of the medical instrument storage system (trolley system) shown in Fig. 1.
Fig. 4 is a block diagram showing a modified example of the medical instrument storage system (trolley system) of the first embodiment according to the present invention.
Fig. 5 is an external perspective view schematically showing the entire configuration of a medical instrument storage system (trolley system) of a second embodiment according to the present invention.
Fig. 6 is a block diagram primarily showing the electrical structure in the medical instrument storage system (trolley system) shown in Fig. 5.
Fig. 7 is a block diagram of a modified example of the medical instrument storage system (trolley system) of the second embodiment according to the present invention.

### Best mode for carrying out the invention

Preferred embodiments according to the present invention will be described referring to the drawings.

### (First Embodiment)

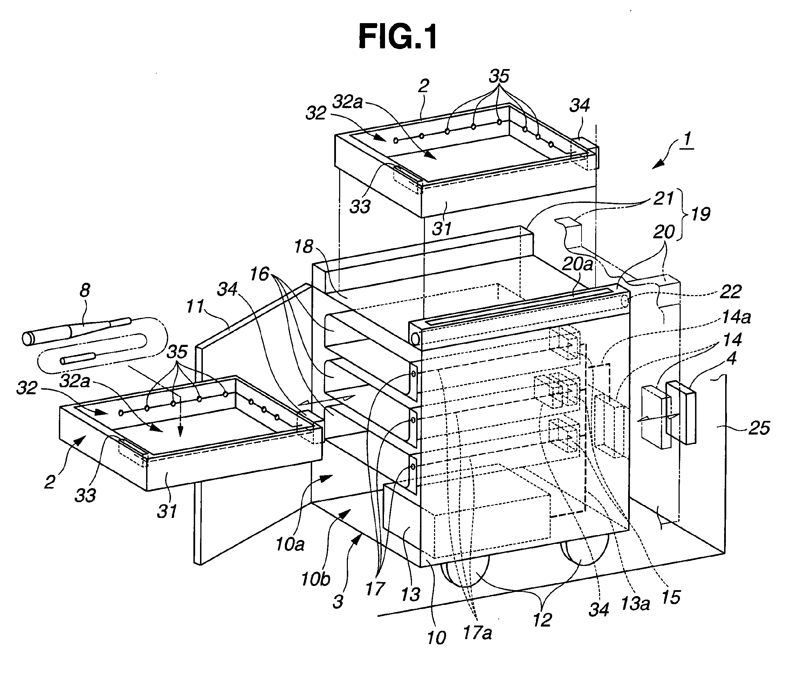

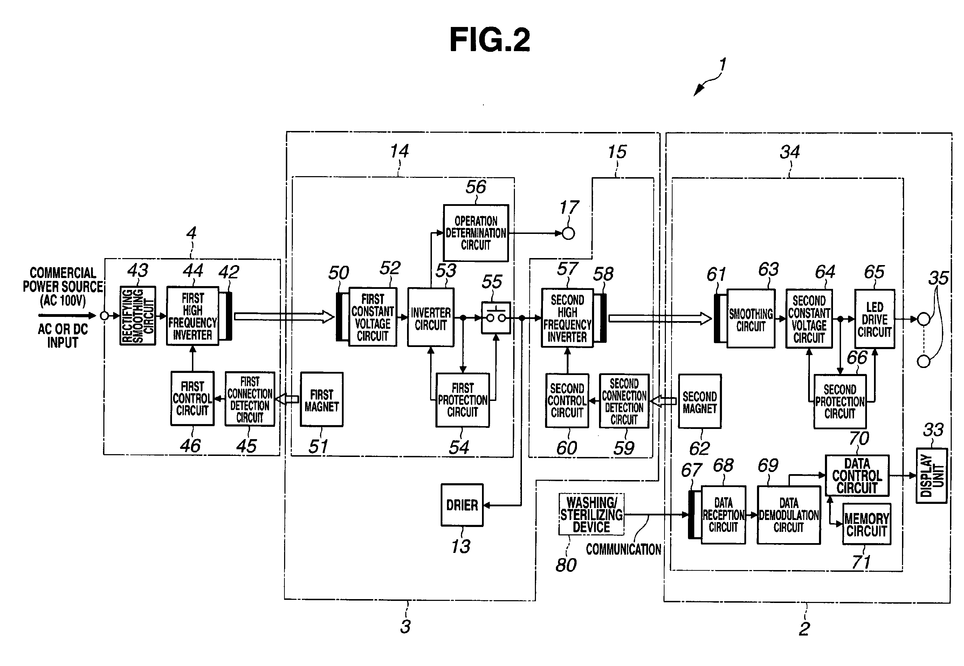

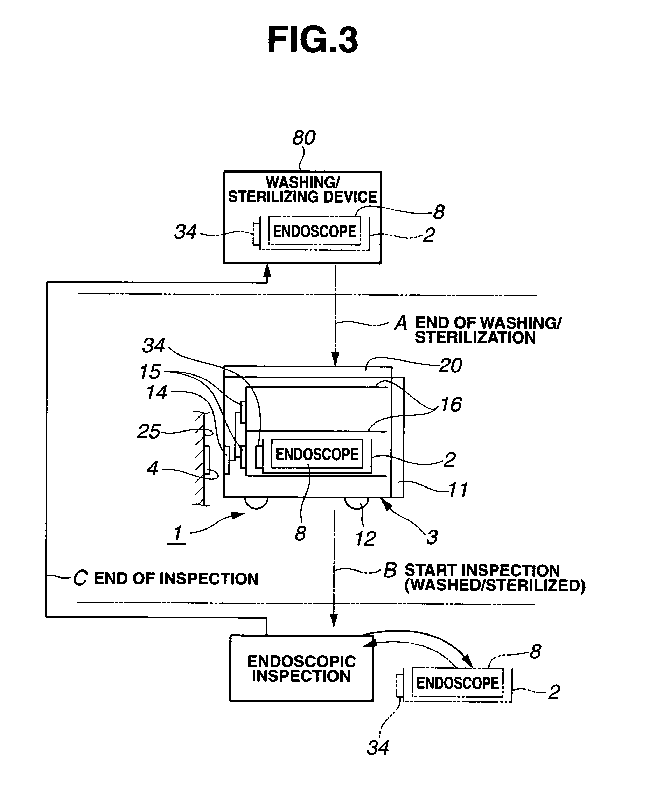

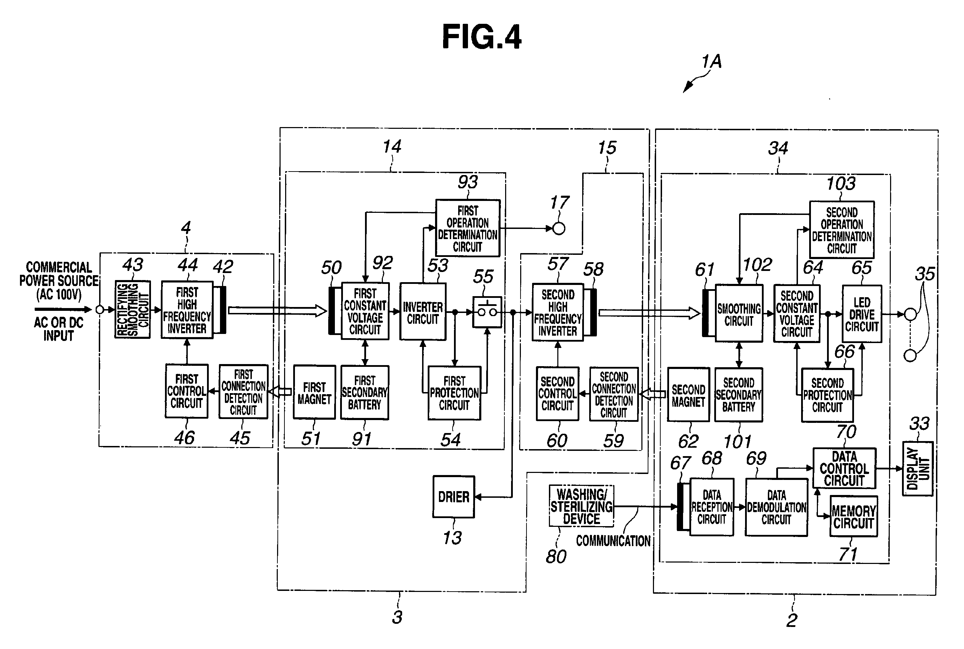

Fig. 1 is an external perspective view showing the entire structure of a medical instrument storage system (trolley system) of a first embodiment according to the present invention. Fig. 2 is a block diagram primarily showing the electrical structure in the medical instrument storage system (trolley system) shown in Fig. 2. Fig. 3 is an explanatory view showing the function of the medical instrument storage system (trolley system) shown in Fig. 1. Fig. 4 is a block diagram of a modified example of the medical instrument storage system (trolley system).

Referring to Figs. 1 and 2, a medical instrument storage system 1 (hereinafter referred to as a trolley system) of the present embodiment mainly includes of plural trays 2 structured to store and keep the medical instrument such as the endoscope and the treatment instrument for operations, a trolley unit 3 as a main body of the trolley system 1 which is structured to detachably arrange the trays 2, and a first power feeder 4 for supplying power attached on the wall to be connected to the power source.

Referring to Fig. 2, the first power feeder 4 structured as a unit is provided to supply power to the trolley system 1. The first power feeder 4 mainly includes a rectifying smoothing circuit 43 which rectifies and smoothes the commercial power source to output direct current, a first high frequency inverter 44 which converts the direct current output from the rectifying smoothing circuit 43 into the high frequency signal to be output, a first primary coil 42 as a primary coil which is connected to the first high frequency inverter 44 and electromagnetically coupled with a first secondary coil 50, a first connection detection circuit 45 as a part of detection means (unit) for detecting the state where the electromagnetic coupling between a first power receiver 14 and the first power feeder 4 (that is, the first power receiver 14 is brought to be close to the first power feeder 4) upon reception of the magnetic force applied by a first magnet 51, and a first control circuit 46 which executes the output control of the first high frequency inverter 44 by constantly monitoring the electromagnetic coupling state.

The first control circuit 46 executes the control to allow/inhibit the first high frequency inverter 44 to output by electromagnetically coupling the first primary coil 42 and the first secondary coil 50 in accordance with the detection result of the first connection detection circuit 45. The first secondary coil 50 outputs the signal to a first constant voltage circuit 52 in response to the high frequency signal transmitted from the first primary coil 42 of the first power feeder 4.

The first power feeder 4 is disposed on a wall surface 25 of the facility, for example, the inspection room where the trolley system 1 is used and the storage facility permanently, for example. The first power feeder 4 is connected to a predetermined power supply for supplying power to the trolley system 1, for example, the generally employed commercial power source.

The first power feeder 4 does not have to be permanently connected to the wall surface 25 but may be installed in the movable unit, for example, another trolley unit. The first power feeder 4 does not have to be placed on the wall surface 25 but may be placed on the floor surface of the inspection room, the storage place or the like. The predetermined power source connected to the first power feeder 4 does not have to be the commercial power source but may be DC power source such as the secondary battery.

The trolley unit 3 serving as the cabinet serves to keep the medical instrument stored in the tray 2 in the sanitary condition. The trolley unit 3 mainly includes a trolley body 10 having an opening 10a in the front surface, a door 11 for opening/closing the opening 10a, four casters 12 each serving as moving means (unit) attached to the bottom of the trolley body 10 for making the trolley unit 3 movable, a drier 13 serving as both sanitary keeping means (unit) and dehumidifying means (unit), the first power receiver 14 serving as power receiver which receives contactless power supply from the first power feeder 4, a second power feeder 15 for supplying power to a second power receiver 34 in the tray 2 (to be described below) in the contactless manner, a wrapping assisting mechanism and the like.

The first power receiver 14 is disposed at a predetermined position on the back surface of the trolley body 10 of the trolley unit 3, that is, the position opposite the first power feeder 4 so as to be directed outward. Referring to Fig. 2, the first power receiver 14 includes a first secondary coil 50 as the secondary coil electromagnetically coupled with the first primary coil 42 of the first power feeder 4, a first constant voltage circuit 52 for smoothing and stabilizing upon reception of the high frequency signal from the secondary coil 50, an inverter circuit 53 for converting the constant voltage output from the first constant voltage circuit 52 into the specified voltage, an output control circuit 55 for outputting the output from the inverter circuit 53 to the drier 13 and the plural second power feeders 15 each serving as the load, a first protection circuit 54 which detects the overcurrent flowing to the load such as the drier 13, and executes the predetermined control, a first magnet 51 which forms a part of the detection means (unit) for detecting the state where the first power receiver 14 is brought to be close to the first power feeder 4 upon movement of the trolley unit 3 together with the output control circuit 55 for controlling the first protection circuit 54, and an operation determination circuit 56 which determines the operation state of the first power receiver 14 and the like.

When the first power receiver 14 is positioned to face the first power feeder 4 as described above by moving the trolley unit 3, the first power receiver 14 receives contactless power supply from the first power feeder 4.

When the trolley unit 3 is brought to be close to the first power feeder 4, and the first power receiver 14 of the trolley unit 3 reaches the predetermined range of the distance from the first power feeder 4, a first connection detection circuit 45 at the first power feeder 4 detects the first magnet 51 and the first power feeder 4 supplies power to the first power receiver 14 in the contactless manner.

The embodiment employs three second power feeders 15 and three second power receivers 34, respectively as shown in Fig. 1. Each of the three second power feeders 15 has the same structure, and each of the three second power receivers 34 has also the same structure. Fig. 2 shows each one of the second power feeders 15 and the second power receivers 34 for simplifying the explanation.

The fundamental of the contactless power supply process between the first power feeder 4 and the first power receiver 14 is structured to be able to separate the first primary coil 42 of the inverter trans from the first secondary coil 50 in the switching power supply, and to convert the output from the predetermined power supply into the high frequency signal for supplying power by electromagnetically coupling the first primary coil 42 with the first secondary coil 50.

The fundamental of the contactless power feeding process between the second power feeder 15 and the second power receiver 34 is the same as described above.

The first connection detection circuit 45 of the present embodiment serves to detect whether or not the power supply through electromagnetic coupling between the first power feeder 4 and the first power receiver 14 is enabled, that is, they are appropriately connected. The first connection detection circuit 45 detects the connection state when the first magnet 51 disposed at the first power receiver 14 side approaches the predetermined distance range. In other words, the first connection detection circuit 45 and the first magnet 51 have the function for detecting the electromagnetic coupling.

For example, in the embodiment, when it is determined that the first power feeder 4 and the first power receiver 14 are appropriately connected, the first control circuit 46 executes the control to drive the first high frequency inverter 44 such that the first primary coil 42 is electromagnetically coupled with the first secondary coil 50.

The inverter circuit 53 serves to convert the constant voltage output from the first constant voltage circuit 52 into the specified AC voltage or the DC voltage. The output signal of the inverter circuit 53 becomes the output from the first power receiver 14 via the output control circuit 55. Either the AC or the DC is applied as the load to the output of the first power receiver 14. The inverter circuit 53 is connected to the operation determination circuit 56 which determines with respect to the operation state of the first power receiver 14.

The operation determination circuit 56 connected to an operation confirmation LED 17 determines whether or not the first power receiver 14 has been normally operated. Switching of the operation confirmation LED 17 is controlled depending on the determination result. If it is determined that the first power receiver 14 has been normally operated, the operation determination circuit 56 serves as the notification means (unit) for notifying the determination result by turning the operation confirmation LED 17 on.

The first protection circuit 54 serves to monitor the connected load state based on the output of the inverter circuit 53.

The output control circuit 55 controls to cut or limit the output of the inverter circuit 53 depending on the detection result of the first protection circuit 54. The output control circuit 55 which exhibits the output protecting function is electrically coupled with the second power feeder 15 and the drier 13.

When the trolley unit 3 is moved such that the first power receiver 14 is brought into the position opposite the first power feeder 4 as described above, the first power receiver 14 receives the contactless power supply from the first power feeder 4.

That is, in the case where the trolley unit 3 is moved to be close to the first power feeder 4 such that the first power receiver 14 of the trolley unit 3 approaches the range of the predetermined distance from the first power feeder 4, the first connection detection circuit 45 at the first power feeder 4 side detects the first magnet 51 to allow the first power feeder 4 to supply power to the first power receiver 14 in the contactless manner.

The second power feeder 15 is configured as a unit to supply power from the power supply to the tray 2 via the first power feeder 4 and the first power receiver 14 in the contactless manner.

The second high frequency inverter 57 connected to the output control circuit 55 of the first power receiver 14 is provided for converting the output signal from the output control circuit 55 into the high frequency signal.

The second primary coil 58 connected to the second high frequency inverter 57 forms an electric power transmission path through which the power supplied from the power supply is fed from the trolley unit 3 to the tray 2 in the contactless manner via the first power feeder 4 and the first power receiver 14 through the electromagnetic coupling with the second secondary coil 61 of the tray 2.

The second connection detection circuit 59 forms detection means (unit) which detects the magnetic force of the second magnet 62 of the second power receiver 34 of the tray 2 to determine whether or not the (second power receiver 34 of) tray 2 has been brought to be close to the second power feeder 15, whether or not the electromagnetic coupling between the second power feeder 15 of the trolley unit 3 and the second power receiver 34 of the tray 2 is enabled, or the connection state between the second power feeder 15 and the second power receiver 34.

Likewise the first control circuit 46, the second control circuit 60 executes the control by determining whether or not the second high frequency inverter 57 is driven in accordance with the detection state of the second connection detection circuit 59.

A storage space 10b communicated with the opening 10a is formed in the trolley body 10 of the trolley unit 3. The opening 10a may be opened and closed by the door 11. When the door 11 is closed, the storage space 10b may be blocked from outside air.

The drier 13 serving as the dehumidifying means (unit) is disposed on the bottom surface inside the storage space 10b*.* The drier 13 dehumidifies air inside the storage space 10b so as to prevent bacteria proliferation in the storage space 10b.

As described later, the storage space 10b is provided with a storage case 16 (holding portion) which detachably holds the tray 2 for storing the medical instrument. The drier 13 as the dehumidification means (unit) further serves as a sanitary maintaining means (unit) for keeping the medical instrument stored in the tray 2 in the sanitary state by humidifying inside of the storage space 10b.

The inner electric circuit (not shown) of the drier 13 is electrically coupled with the first power receiver 14 (reference numeral 13a shown in Fig. 1) so as to be driven by the power fed from the first power feeder 4 via the first power receiver 14.

A shelf structure is formed in the predetermined inner area of the storage space 10b. That is, plural stacked storage cases 16 (three cases as shown in Fig. 1) as the tray holding portion are fixed above the position on which the drier 13 is disposed.

Each of the storage cases 16 has an opening in the front surface through which the tray 2 is inserted so as to be stored therein. The tray 2 is kept at a predetermined position in the trolley body 10 of the trolley unit 3. The storage case 16 of the trolley body 10 serves as the holding portion for holding the medical instrument while holding the tray 2.

The surface opposite the opening in each of the respective storage cases 16 is provided with the second power feeder 15.

Referring to Fig. 2, the second power feeder 15 is provided for feeding power from the power source to the tray 2 via the first power feeder 4 and the first power receiver 14 in the contactless manner. The second power feeder 15 mainly includes a second high frequency inverter 57 which converts the output from the output control circuit 55 of the first power receiver 14 into the high frequency signal, a second primary coil 58 which is connected to the second high frequency inverter 57 so as to be electromagnetically coupled with the second secondary coil 61, a second connection detection circuit 59 as a part of the detection means (unit) for detecting that the electromagnetic coupling between the second power feeder 15 and the second power receiver 34 of the tray 2 is enabled upon reception of the magnetic force of the second magnet 51 of the tray 2 (the tray 2 (second power receiver 34) has been brought to be close to the second power feeder 15), and a second control circuit 60 which constantly monitors the electromagnetic coupling state to execute output control of the second high frequency inverter 57.

The second control circuit 60 electromagnetically couples the second primary coil 58 with the second secondary coil 61 depending on the detection result of the second connection detection circuit 59 for executing the control to allow/inhibit the output of the second high frequency inverter 57.

Each of the second power feeders 15 for the storage case 16 corresponding to the tray 2 is electrically coupled with the first power receiver 14, respectively (see reference numeral 14a shown in Fig. 1).

A light-emitting diode (hereinafter referred to as LED) 17 for confirmation of the operation is disposed at a predetermined position around the peripheral edge of the opening at the front surface side of each of the storage cases 16. The LED 17 for confirmation of the operation is electrically coupled with the first power receiver 14 as described above (see the reference numeral 17a shown in Fig. 1).

A tray mount portion 18 is provided on the upper surface of the trolley body 10 of the trolley unit 3, on which a wrapping assisting mechanism 19 formed of a wrapping storage member 20 and a limiting member 21 is mounted. The wrapping assisting mechanism 19 includes the wrapping storage member 20 and the limiting member 21 each formed as a substantially prism-like wall serving to limit the lateral movement of the tray 2 mounted on the tray mount 18 as the limiting means (unit).

The wrapping storage member 20 and the limiting member 21 extend from the front to the back surface of the trolley body 10 substantially in parallel with each other. The wrapping storage member 20 and the limiting member 21 are disposed at an interval which is substantially the same as the width of the tray 2, or slightly larger than the width of the tray 2.

A roll wrapping paper 22 as the wrapping member is stored inside the wrapping storage member 20. As the wrapping paper 22, a water absorption sheet or a dust-proof sheet may be employed.

A wrapping opening 20a is formed in the upper surface of the wrapping storage member 20 through which the sheet of the wrapping paper 22 is pulled out. Meanwhile, a blade and the like (not shown) is attached to the limiting member 21 for cutting the sheet of the wrapping paper 22.

The wrapping storage member 20 and the limiting member 21 which form the wrapping assisting mechanism 19 have substantially prism-like walls. The wall functions as the limit means (portion) to limit the lateral movement of the tray 2 mounted on the tray mount portion 18.

As described above, the four casters 12 are attached to the bottom surface of the trolley body 10 of the trolley unit 3. The casters 12 serve as the moving means (unit) for moving the trolley unit 3 on the floor surface.

The tray 2 is arranged in the trolley unit 3 so as to be used to hold the medical instrument, and to carry the medical instrument separated from the trolley unit 3.

The outer shape of the tray 2 mainly includes a substantially rectangular solid case body 31 having an opening 32a in one surface, a storage portion 32 formed by the case body 31 as the holding portion, a display unit 33 for displaying predetermined information, the second power receiver 34 which receives contactless power supply from the second power feeder 15, and the sterilizing lamp 35 as the sterilizing means (unit) and the like.

The case body 31 has a storage portion 32 with the opening 32a formed in the upper surface. The storage portion 32 is structured to store the predetermined medical instrument, for example, an endoscope 8.

Plural sterilizing lamps 35 each as the sterilizing means (unit), that is, the sanitary keeping means (unit) for keeping the sanitary state of the medical instrument stored in the tray 2 are disposed on the inner wall surface of the storage portion 32 while preventing the bacteria proliferation in the trolley unit 3 or the tray 2. For example, the mercury lamp or the UV-LED as the UV light source for emitting the UV light may be used as the sterilizing lamp 35.

In the present embodiment, the plural sterilizing lamps 35 are arranged at predetermined intervals on the inner wall surface of the storage portion 32 of the tray 2 as shown in Fig. 1. However, arrangement or the number of the sterilizing lamps 35 may not be limited to the one as shown in the drawing.

The second power receiver 34 is disposed and directed outward at the predetermined position on the back surface of the case body 31 of the tray 2. The second power receiver 34 is positioned to face the second power feeder 15 when the tray 2 is set in the storage case 16 of the trolley unit 3. When the second power receiver 34 is brought to be close to the predetermined range of the distance from the second power feeder 15 by setting the tray 2 in the storage case 16, the second power receiver 34 receives the contactless power supply from the second power feeder 15.

The second power receiver 34 is provided with the second secondary coil 61, the second magnet 62 which forms a part of the detection means (unit), a smoothing circuit 63, a second constant voltage circuit 64, an LED drive circuit 65, a second protection circuit 66, a reception coil 67 and a data reception circuit 68, a data demodulation circuit 69, a memory circuit 71 which contains the flash memory, for example, a data control circuit 70 for controlling the information management, and the like.

The second secondary coil 61 receives the high frequency signal which has been transmitted from the second primary coil 58 of the second power feeder 15 through the electromagnetic coupling so as to be output to the smoothing circuit 63. The smoothing circuit 63 smoothes the output of the second secondary coil 61.

The second constant voltage circuit 64 stabilizes the output of the smoothing circuit 63. The second constant voltage circuit 64 is connected to the LED drive circuit 65 and the second protection circuit 66 at the output side.

The second protection circuit 66 monitors the connected load state based on the output of the second constant voltage circuit 64 so as to function in protecting the circuit when the overload is applied.

Upon reception of the output of the second constant voltage circuit 64, the LED drive circuit 65 lights the plural sterilizing lamps 35 on. As shown in Figs. 1 and 2, the LED drive circuit 65 and the sterilizing lamps 35 may be disposed inside the tray 2, or inside the storage case 16 of the trolley unit 3.

The tray 2 formed as a unit with the water-proof structure is disposed in a washing/sterilizing device 80 (see Fig. 2) so as to use the tray 2 for washing/sterilizing the medical instrument.

In the aforementioned case, the history information such as the result of washing/sterilizing may be obtained through the wireless data communication between the tray 2 and the washing/sterilizing device 80. The history information is displayed on the display unit 33 arranged on the upper surface of the front wall of the tray 2, for example. In the state where the tray 2 is set in the storage case 16 of the trolley unit 3, the display unit 33 is exposed such that the display screen is visually confirmed.

Meanwhile, the tray 2 disposed inside the separately provided washing/sterilizing device 80 (see Fig. 2) may be used as the one for washing/sterilizing the medical instrument. In this case, the washing/sterilizing device 80 is configured including the power feeder corresponding to the second power feeder 15, a transmission coil, and a data transmission circuit (not shown). The contactless power supply is allowed from the power feeder of the washing/sterilizing device 80 to the second power receiver 34 of the tray 2 through the electromagnetic coupling. This makes it possible to supply power to the tray 2 disposed inside the washing/sterilizing device 80.

The data communication is performed between the data reception circuit 68 of the tray 2 and the data transmission circuit (not shown) of the washing/sterilizing device 80 via the reception coil 67 and the transmission coil (not shown) at the washing/sterilizing device 80. In the case where the endoscope 8 is washed/sterilized in the washing/sterilizing device 80 on the tray 2, the data reception circuit 68 receives the history information with respect to the endoscope 8.

The data demodulation circuit 69 demodulates the history information from the data reception circuit 68 under the control of the data control circuit 70.

The data control circuit 70 is structured to write /read the demodulated history information data into/from the memory circuit 71. The flash memory installed in the memory circuit 71 is structured to maintain the information which has been written even if the power supply from the second power receiver 34 is cut.

The display unit 33 displays the history information under the control of the data control circuit 70. The history information includes the processing result information indicating whether or not the process has been normally executed, the information of the processing date and ID information on the medical instrument subjected to the processing.

The operation using the endoscope 8 as the medical instrument in the above-structured trolley system 1 of the present embodiment will be described referring to Figs. 1 to 3.

It is assumed that the first power feeder 4 is disposed apart from the trolley unit 3 at a predetermined distance as shown by the solid line in Fig. 1, and the tray 2 which holds the endoscope 8 is set in the storage case 16 inside the trolley unit 3 as shown in Fig. 3. It is also assumed that at least one of the storage cases 16 does not have the tray 2 set therein. In Fig. 3, only two storage cases 16 of the trolley unit 3 are shown, and other case is not shown.

The first power feeder 4 is constantly connected to the commercial power source via the outlet of the wall surface 25. The first connection detection circuit 45 of the first power feeder 4 is apart from the first magnet 51 of the first power receiver 14 by a predetermined distance or longer, and accordingly, detects no magnetic force. Upon reception of the detection signal of the first connection detection circuit 45, the first control circuit 46 stops the output of the first high frequency inverter 44.

The first high frequency inverter 44 does not output the DC smoothed by rectifying the commercial power source by the rectifying smoothing circuit 43. The components other than the first high frequency inverter 44 are activated to be automatically brought into the stand-by state. Accordingly, there is no output from the first primary coil 42. The rectifying smoothing circuit 43 of the embodiment converts the AC input as the commercial power source supplied from the outlet (not shown) of the wall surface 25 into the DC. In this case, either AC or DC may be input to the power source.

The first high frequency inverter 44 of the embodiment converts the DC signal smoothed by the rectifying smoothing circuit 43 into the high frequency signal such that the output of the high frequency signal is enabled and disabled. The first primary coil 42 converts the high frequency signal from the first high frequency inverter 44 into the electromagnetic energy so as to be transmitted to the first secondary coil 50 of the first power receiver 14.

The electromagnetic coupling between the first secondary coil 50 of the first power receiver 14 of the trolley unit 3 and the first primary coil 42 of the first power receiver 14 is interrupted. Power is not supplied to the first power receiver 14 of the trolley unit 3 and the second power receiver 34 of the tray 2. The drier 13 is stopped, and the sterilizing lamps 35 are kept turned off. As the LED 17 for confirming the operation is also kept turned off, the user is allowed to open the door 11 of the trolley unit 3 to confirm that no power has been supplied to the trolley unit 3.

The trolley unit 3 as the cabinet in the state that the door 11 kept closed keeps the endoscope 8 on the tray 2 in the storage space 10b in the sanitary state while screening out the dust.

The trolley unit 3 is moved by the casters 12 such that the back surface of the trolley unit 3 is brought to be close to the first power feeder 4 as indicted by the two-dot chain line of Fig. 1, and Fig. 2. Then the first power receiver 14 of the trolley unit 3 comes close to the predetermined range of the distance from the first power feeder 4 such that the first connection detection circuit 45 receives the magnetic force of the first magnet 51. Then the first connection detection circuit 45 detects that the electromagnetic coupling between the first power receiver 14 and the first power feeder 4 is enabled.

Upon reception of the detection signal from the first connection detection circuit 45, the first control circuit 46 allows the first high frequency inverter 44 to output. The first high frequency inverter 44 converts the DC output from the rectifying smoothing circuit 43 into the high frequency signal so as to be supplied to the first primary coil 42. The first control circuit 46 constantly monitors the electromagnetic coupling condition, and allows the first high frequency inverter 44 to output as long as the appropriate coupling state is established.

The high frequency signal is transmitted from the first primary coil 42 to the first secondary coil 50 of the first power receiver 14 for performing the contactless power supply. Upon reception of the power, the respective circuits in the first power receiver 14 are activated.

In the first power receiver 14, the high frequency signal from the first secondary coil 50 is supplied to the first constant voltage circuit 52 for smoothing and stabilizing the high frequency signal. The inverter circuit 53 converts the constant voltage output from the first constant voltage circuit 52 into the specified voltage. The output control circuit 55 outputs the output from the inverter circuit 53 to the drier 13 and the plural second power feeders 15 at the load side.

As the drier 13 is activated, the inside of the storage space 10b of the trolley unit 3 is dehumidified to suppress the bacteria proliferation, and to keep the endoscope 8 stored in the tray 2 in the sanitary state.

The operation determination circuit 56 connected to the inverter circuit 53 is also operated to light the operation confirmation LED 17 at the side of the storage case 16. The user is allowed to determine if power is appropriately supplied to the trolley unit 3 by visually confirming lighting of the operation confirmation LED 17.

Meanwhile, when the trolley unit 3 is moved to be away from the first power feeder 4 on the wall surface 25 by a predetermined distance or longer, the magnetic force of the first magnet 51 becomes ineffective. Then the first connection detection circuit 45 of the first power feeder 4 detects interruption of the electromagnetic coupling. Upon reception of the detection signal of the first connection detection circuit 45, the first control circuit 46 causes the first high frequency inverter 44 to stop outputting. When the trolley unit 3 is separated, the first power feeder 4 on the wall surface 25 stops the power supply. As described above, the trolley system 1 of the embodiment may be safely used constantly since the first power feeder 4 is automatically brought into the stand-by state.

When failure occurs in the drier 13, for example to apply the overcurrent to the load side in the operative state of the first power feeder 14, the first protection circuit 54 is activated to control the output of the output control circuit 55. The output control circuit 55 is operated to interrupt the output to stop the drier 13, the sterilizing lamps 35, and the display unit 33. The interruption of the output functions in preventing the overcurrent to flow into the first power receiver 14 to protect the circuit from failure or the like.

The operation of the trolley system 1 of the present embodiment for washing/sterilizing the endoscope 8 and performing the endoscopic inspection will be described hereinafter.

It is assumed that the place where the first power feeder 4 is disposed on the wall surface 25, that is, the place for storing the trolley unit 3 is different from the place for installing the washing/sterilizing device 80. It is also assumed that the inspection room for performing the endoscopic inspection is different from the aforementioned storage place and the installation place.

First, at the place where the washing/sterilizing device 80 is installed, when the operation for washing and sterilizing the endoscope 8 is finished, data communication is performed between the washing/sterilizing device 80 and the tray 2 attached thereto. The second power receiver 34 of the tray 2 receives contactless power supply from the power feeder of the washing/sterilizing device 80 through the electromagnetic coupling.

The history information is transmitted from the data transmission circuit of the washing/sterilizing device 80 to the data reception circuit 68 of the tray 2 via the transmission coil and the reception coil 67. The data demodulation circuit 69 of the tray 2 demodulates the history information from the data reception circuit 68 under the control of the data control circuit 70 which in turn writes the demodulated history information into the memory circuit 71.

Next, referring to a code A shown in Fig. 3, the tray 2 that stores the endoscope 8 is taken out from the washing/sterilizing device 80 so as to be carried to the storage place of the trolley unit 3 while keeping the cleaned endoscope 8 in the storage state.

The tray 2 is then set on the tray mount portion 18 of the trolley unit 3. As the movement of the tray 2 is limited by the wrapping storage member 20 and the limiting member 21, detachment of the tray resulting from the lateral displacement may be prevented.

The roll wrapping paper 22 is withdrawn from the wrapping opening 20a of the wrapping storage member 20 to perform wrapping to seal the opening 32a of the tray 2. The inside of the storage portion 32 of the tray 2, thus, may be protected from the dust and the like.

The aforementioned operation may be performed at the place around the washing/sterilizing device 80 by moving the trolley unit 3 as necessary.

At the place for storing the trolley 3, the first power receiver 14 of the trolley unit 3 is connected to the first power feeder 4 on the wall surface 25 so as to supply power to the first power receiver 14. Upon reception of the power supply, the operation confirmation LED 17 is lit. The user at this time is allowed to confirm if the power is appropriately supplied by opening the door 11 of the trolley unit 3 to check lighting of the operation confirmation LED 17.

As the drier 13 is operated, the inside of the storage space 10b of the trolley unit 3 is dehumidified, thus suppressing the proliferation of bacteria and the like.

The user is then allowed to set the wrapped tray 2 into the open space of the storage case 16, and to close the door 11 so that the inside of the storage space 10b of the trolley unit 3 is sealed. Therefore, the endoscope 8 stored on the wrapped tray 2 which has been washed/sterilized may be kept in the sanitary storage state.

When the tray 2 is set in the storage case 16, the second power receiver 34 of the trolley unit 3 faces the second power feeder 15 of the trolley unit 3 at a predetermined distance. Then, the electromagnetic force of the second magnet 62 becomes active to allow the second connection detection circuit 59 to detect the state where power supply through the electromagnetic coupling is enabled, and to output the predetermined detection signal.

Upon reception of the detection signal, the second control circuit 60 allows the second high frequency inverter 57 to output. The second high frequency inverter 57 converts the output from the output control circuit 55 into the high frequency signal. The second control circuit 60 constantly monitors the electromagnetic coupling state, and allows the second high frequency inverter 57 to output so long as the connection is in the appropriate state.

The second primary coil 58 which has received the high frequency signal is electromagnetically coupled with the second secondary coil 61 to perform the contactless power supply from the second power feeder 15 to the second power receiver 34. The power generated in the second secondary coil 61 is supplied to the LED drive circuit 65 and the display unit 33 at the load side via the smoothing circuit 63 and the second constant voltage circuit 64.

Upon reception of the power supply, the LED drive circuit 65 lights the plural sterilizing lamps 35 disposed on the trays 2. The UV rays for photocatalytic and sterilizing purpose are irradiated to the photocatalytic portion 37 of the tray 2 such that the antibacterial and antifouling effects of the photocatalyst act on the photocatalytic portion 37.

The outer portion of the tray 2 and the inner surface of the storage portion 32 where the photo catalytic portion 37 is formed may be in the sanitary stain-resistant state. Moreover, the UV rays irradiated from the sterilizing lamps 35 prevent the growth and proliferation of bacteria. This makes it possible to keep the tray 2 and the endoscope 8 in the sanitary state.

As the typical photocatalytic material, titanium oxide may be employed. However, arbitrary material may be employed so long as the equivalent function and performance are obtained. Generally, the photocatalytic material absorbs UV ray, promotes the chemical reaction with the other substance without itself being changed, and performs the environmental purification, for example, antifouling, air purification, antibacterial, and antifugal functions.

In other words, the photocatalytic portion 37 serves to allow the antibacterial function, antifouling function and the like to be effectively performed upon reception of the UV rays from the sterilizing lamps 35. Therefore, the UV rays emitted from the UV light source also provide the sterilizing effect for protection against the proliferation of bacteria and the like.

The sterilizing lamp 35 is structured to irradiate the light to the medical instrument stored in the storage portion 32 and the photocatalytic portion 37. The mercury lamp, UV-LED and the like serving as the UV light source for emitting UV rays are applied to the sterilizing lamp 35. The light from the sterilizing lamp 35 is also irradiated to the photocatalytic portion 37. Therefore, the photocatalytic portion 37 receives the light irradiated from the sterilizing lamp 35, allowing the photocatalytic member to perform its function.

In the second power feeder 15, the power supplied via the second secondary coil 61 is fed to the display unit 33 and the data control circuit 70. The data control circuit 70 activated upon reception of the power supply reads the history information such as the last washing/sterilizing result stored in the memory circuit 71. The display unit 33 displays the history information read by the data control circuit 70 from the memory circuit 71. The user visually checks the history information displayed on the display unit 33 to confirm the history information of the endoscope 8 stored in the tray 2, that is, the processing results, processing date and the type of the endoscope.

In the state where the second power receiver 34 is operated, upon detection of the failure which occurs in the sterilizing lamp 35, or overcurrent applied to the load side, the second protection circuit 66 interrupts the output of the second constant voltage circuit 64 so as to prevent the overcurrent from flowing into the second power receiver 34. This makes it possible to prevent failure in the circuit. The sterilizing lamp 35 is turned off, and the display unit 33 stops displaying.

The user confirms the ID information, the washing/sterilizing result, and the washing/sterilizing date with respect to the endoscope 8 displayed on the display unit 33 of the tray 2 for the endoscopic inspection or the operation. This makes it possible to select the desired endoscope or the medical instrument from the plural medical instruments such as the endoscope stored in the trolley unit 3.

The user takes out the tray 2 which holds the washed and sterilized endoscope 8 from the trolley unit 3 in accordance with the inspection to be executed. When the tray 2 is detached, the distance between the second power feeder 15 and the second power receiver 34 becomes longer than the predetermined distance such that those components are disconnected. The magnetic force of the second magnet 62 becomes ineffective, which is detected by the second connection detection circuit 59.

Upon reception of the detection signal of the second connection detection circuit 59, the second control circuit 60 interrupts output of the second high frequency inverter 57. That is, the second power feeder 15 of the trolley unit 3 is automatically brought into the stand-by state to cut the power supply to the second power receiver 34 of the tray 2. In the tray 2 with the second power receiver 34 to which the power is no longer supplied, the plural sterilizing lamps 35 are turned off, and the display unit 33 stops displaying.

The wrapped tray 2 which holds the endoscope 8 is carried to the inspection room.

Referring to a code C shown in Fig. 3, after the predetermined inspection is finished, the used endoscope 8 held on the tray 2 is carried to the place where the washing/sterilizing device 80 is installed.

The user sets the tray 2 which holds the used endoscope 8 in a cleaning vessel of the washing/sterilizing device 80 so as to be subjected to a series of the washing/sterilizing process performed by the washing/sterilizing device 80.

Referring to the code A of Fig. 3, when the washing/sterilizing process is finished, the tray 2 is taken out from the washing/sterilizing device 80, and wrapped as described above. Thereafter, the user sets the tray 2 which holds the endoscope 8 cleaned in the washing/sterilizing process in the storage case 16 of the trolley unit 3. This makes it possible to keep the endoscope 8 which has been washed and sterilized in the sanitary state in the trolley unit 3.

In the aforementioned embodiment, the trolley unit 3 is made movable by means of the casters 12 such that the first power feeder 4 connected to the predetermined power source is brought to be close to face the first power receiver 14 of the trolley unit 3. The contactless power supply from the first power feeder 4 to the first power receiver 14 is performed through electromagnetic coupling therebetween such that the trolley unit 3 receives the required power supply. In the aforementioned case, specifically, the power is supplied to the respective circuits of the first power receiver 14, the drier 13, and the tray 2.

In response to the power supply, the drier 13 as the dehumidifying unit dehumidifies the inside of the storage space 10b of the trolley unit 3 so as to keep the stored medical instrument in the sanitary state.

The trolley unit 3 structured to be movable by the casters 12 stores the medical instrument inside the trolley body 10, and allows the first power receiver 14 and the first power feeder 4 to be easily separated. This makes it possible to move the trolley unit 3 while keeping the medical instrument therein.

That is, the user is allowed to carry the medical instrument without being taken out from the trolley unit 3. Unlike the conventional device with the power cord, as the trolley unit 3 requires no power cord, insertion of the power cord is not required for the movement. This makes it possible to move the device easily.

When the user sets the tray 2 in the storage case 16 disposed in the trolley body 10 of the trolley unit 3, the second power receiver 34 is brought to be close to face the second power feeder 15 to which the power is applied from the first power feeder 4. Thus, the contactless power transmission is performed from the second power feeder 15 to the second power receiver 34 such that the tray 2 receives the required power supply. The power is further supplied to the sterilizing lamp 35 such as the UV-LED serving as the sterilizing means (unit) of the tray 2 to light the sterilizing lamp 35 to emit the UV rays. The UV light rays are used for sterilizing the medical instruments stored in the storage portion 32 of the tray 2 to keep the medical instrument in the sanitary environment until the subsequent use.

The tray 2 provided with the second power receiver 34 is structured to be separated from the second power feeder 15 of the trolley unit 3, and to be detachable with respect to the trolley body 10 of the trolley unit 3. Accordingly, the tray 2 is allowed to keep the stored medical instrument in the trolley unit 3. The medical instrument may be easily carried to the desired place such as the inspection room in the sanitary condition only by taking out the tray 2 from the trolley unit 3 when needed for the inspection, for example.

As the trolley system 1 allows the used medical instrument to be carried while being stored in the storage portion 32 of the tray 2, the liquid leakage or stain of the hand of the user may be prevented. The user is allowed to operate the washing/sterilizing device 80 in the sanitary state without staining the device.

The trolley system 1 returns the tray 2 which holds the cleaned medical instrument to the trolley unit 3 after the washing and sterilizing process so as to supply power to the sterilizing lamp 35 and the like of the tray 2 again. This makes it possible to keep the medical instrument in the sanitary state.

The trolley system 1 according to the embodiment carries the medical instrument held on the tray 2, and keeps the medical instrument using on the tray 2 and the trolley unit 3in the sanitary state. The use and management of the medical instrument may be efficiently implemented in accordance with the mode of the use, for example, washing/sterilizing process, inspection and the like.

The plural storage cases 16 of the trolley unit 3 allow the plural medical instruments to be stored simultaneously by setting the respective trays 2. The medical instruments respectively held on the plural trays 2 are carried to the different places such that the predetermined operations are performed independently, resulting in efficient use of the medical instruments.

In the trolley system 1, when the connection detection circuit 59 detects the state where the power supply is enabled, the control circuit 60 which receives the detection signal controls such that the power is output from the power feeder to perform the contactless power supply to the power receiver.

In contrast, upon detection of the state where power is not supplied by the connection detection circuit 59, the control circuit 60 controls such that the output from the power feeder is stopped. Under the aforementioned control, when the power receiver does not exist, the unnecessary power supply is not performed.

In the trolley system 1, the tray 2 is mounted on the upper surface of the trolley unit 3, and the wrapping paper 22 is withdrawn from the wrapping opening 20a of the wrapping storage member 20 so as to seal the opening 32a of the tray 2 with the wrapping paper 22. The aforementioned wrapping process allows the trolley system 1 to prevent intrusion of the bacteria, dust and the like from the opening 32a of the tray 2. This makes it possible to keep the medical instrument in the sanitary state during its carriage and storage.

Next, a modified example of the trolley system of the first embodiment according to the present invention will be described.

Fig. 4 is a block diagram showing the modified example of the trolley system of the first embodiment as described above.

The basic configuration of the modified example is substantially the same as that of the aforementioned embodiment. Accordingly, the same components as those of the embodiment will be designated with the same reference numerals, and the explanation thereof, thus will be omitted. The description with respect to only the difference will be explained.

A trolley system 1A according to the modified example includes a first secondary battery 91 and a second secondary battery 101 each rechargeable inside the first power receiver 14 of the trolley unit 3 and the second power receiver 34 of the tray 2 in addition to the configuration of the embodiment as described above.

Referring to Fig. 4, the first power receiver 14 according to the modified example includes a first operation determination circuit 93 serving as the first constant voltage circuit 92 and the output switching means (unit) instead of the first constant voltage circuit 52 and the operation determination circuit 56 in the aforementioned embodiment.

The first constant voltage circuit 92 of the first power receiver 14 is connected to a rechargeable first secondary battery 91. The first secondary battery 91 is rechargeable by the charging circuit (not shown) built in the first power receiver 14. The first operation determination circuit 93 determines with respect to the operation state of the first power receiver 14.

In the case where appropriate power is supplied to the first power receiver 14, the first constant voltage circuit 92 operates in the same manner as the first constant voltage circuit 52 according to the aforementioned embodiment. In the case where the connection to the first power receiver 14 is interrupted, and the first operation determination circuit 93 detects the state where the output has been stopped, the control for switching the output of the first constant voltage circuit 92 to that of the first secondary battery 91 is executed. Other operations of the first operation determination circuit 93 are the same as those of the operation determination circuit 56 of the aforementioned embodiment.

The second power receiver 34 in the modified example includes a smoothing circuit 102 instead of the smoothing circuit 63 of the aforementioned embodiment. The second power receiver 34 further includes a second operation determination circuit 103 serving as the output switching means (unit).

The smoothing circuit 102 of the second power receiver 34 is connected to a rechargeable second secondary battery 101. The second secondary battery 101 is rechargeable by the charging circuit (not shown) built in the second power receiver 34. The second operation determination circuit 103 monitors the output of the second constant voltage circuit 64 to determine with respect to the operation state of the second power receiver 34.

In the case where the appropriate power is supplied to the second power receiver 34, the smoothing circuit 102 operates in the same manner as the smoothing circuit 63 of the aforementioned embodiment. In the case where the connection in the first power receiver 14 or the second power receiver 34 is interrupted, and the second operation determination circuit 103 detects the state where the output has been stopped, the control for switching the output of the smoothing circuit 102 to that of the second secondary battery 101 is executed.

Other configuration is substantially the same as that of the aforementioned embodiment.

The trolley system 1 of the modified example executes the switching control such that power is supplied from the first secondary battery 91 and the second secondary battery 101 even if the connection between the respective power feeders and power receivers is interrupted, thus temporarily driving the inner circuit.

Therefore, the trolley system 1 of the modified example allows the power supply to the drier 13 of the trolley unit 3 and the tray 2 although the trolley unit 3 is separated from the first power feeder 4 for the purpose of carrying the endoscope 8, for example.

In other words, when the trolley unit 3 is separated from the first power feeder 4, the first operation determination circuit 93 for monitoring the output of the inverter circuit 53 determines the disconnection state, and then executes the control for switching the output of the first constant voltage circuit 92 to that of the first secondary battery 91.

Thus, power of the first secondary battery 91 is supplied to the drier 13 and the second power feeder 15 of the tray 2 via the first constant voltage circuit 92, the inverter circuit 53 and the output control circuit 55. As the second power receiver 34 receives power from the second power feeder 15, the sterilizing lamp 35 for sterilization may be continuously lit.

When the user detaches the tray 2 from the trolley unit 3, the second operation determination circuit 103 for monitoring the output of the second constant voltage circuit 64 determines with respect to transition to the disconnection state. The second operation determination circuit 103 executes the control for switching the output of the smoothing circuit 102 to that of the second secondary battery 101.

Thus, the power from the second secondary battery 101 is supplied to the LED drive circuit 65 via the smoothing circuit 102 and the second constant voltage circuit 64 such that the sterilizing lamp 35 for sterilization is continuously lit by the LED drive circuit 65. The power from the second secondary battery 101 is supplied to the display unit 33 and the data control circuit 70 and the like such that the history information on the medical instrument is kept displayed on the display unit 33.

Although the user takes out the tray 2 from the trolley unit 3, the history information indicating when the endoscope 8 stored in the tray 2 has been washed and cleaned may be confirmed any time. This makes it possible to manage the history information with respect to the washing/sterilizing process.

As described above, the same effects as those of the above described embodiment may be derived from the modified example.

In addition, in the modified example, either the first operation determination circuit 93 or the second operation determination circuit 103 executes the control for switching the output to that of the first secondary battery 91 or the second secondary battery 101 irrespective of no output from the first power receiver 14 or the second power receiver 34. Thus, the trolley system 1 according to the modified example is allowed to constantly supply the required power to the trolley unit 3 and the tray 2.

Therefore, when the trolley unit 3 is separated from the first power feeder 4, the trolley system 1 is capable of supplying power to the tray 2 and the drier 13 even if the trolley unit 3 is moving. As the trolley system 1 is allowed to ensure the power supplied to the sterilizing lamp 35 of the tray 2 or the display unit 33 during carriage of the tray 2, the sterilized state may be constantly maintained, and the history information may be confirmed.

As the display unit 33 is allowed to constantly display the history information with respect to the medical instrument in the carriage state, the history information with respect to the type of the medical instrument, the date when the washing/sterilization has ended may be confirmed any time. Even if the plural trays 2 separated from the trolley unit 3 exist in the inspection room simultaneously, the medical instruments stored inside the respective trays 2 may be easily managed.

### (Second embodiment)

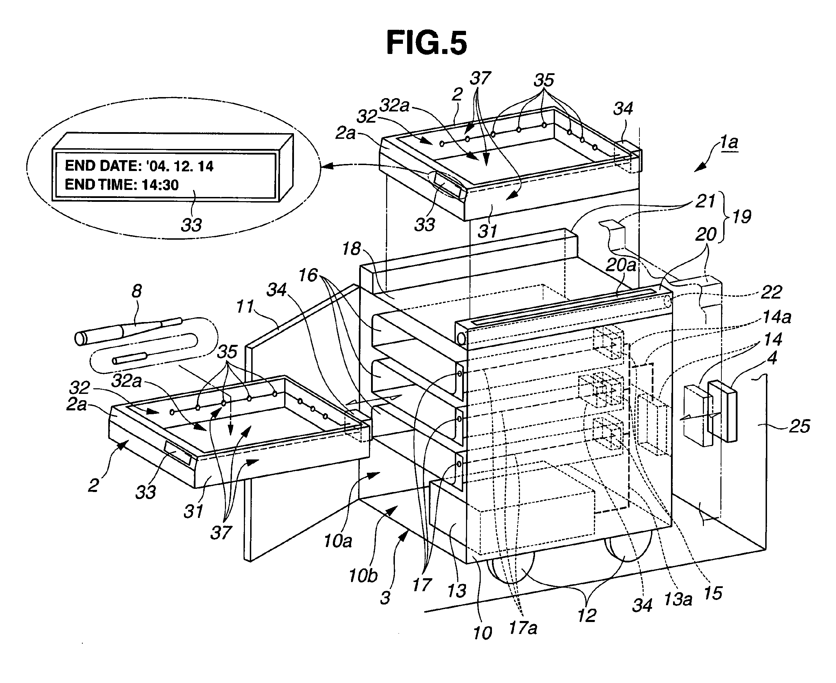

A second embodiment according to the present invention will be described referring to Figs. 5 to 7.

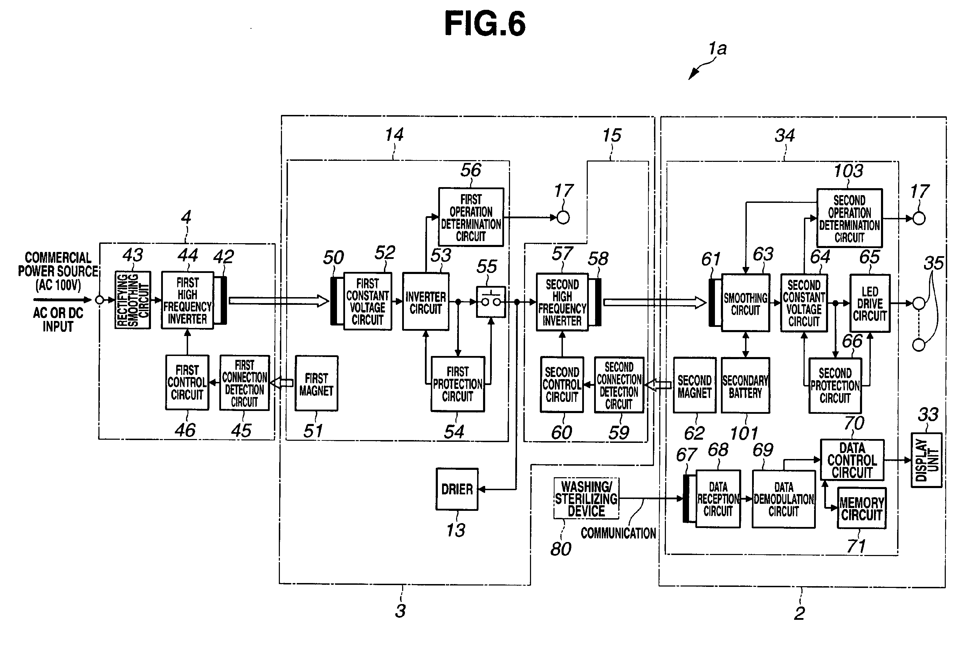

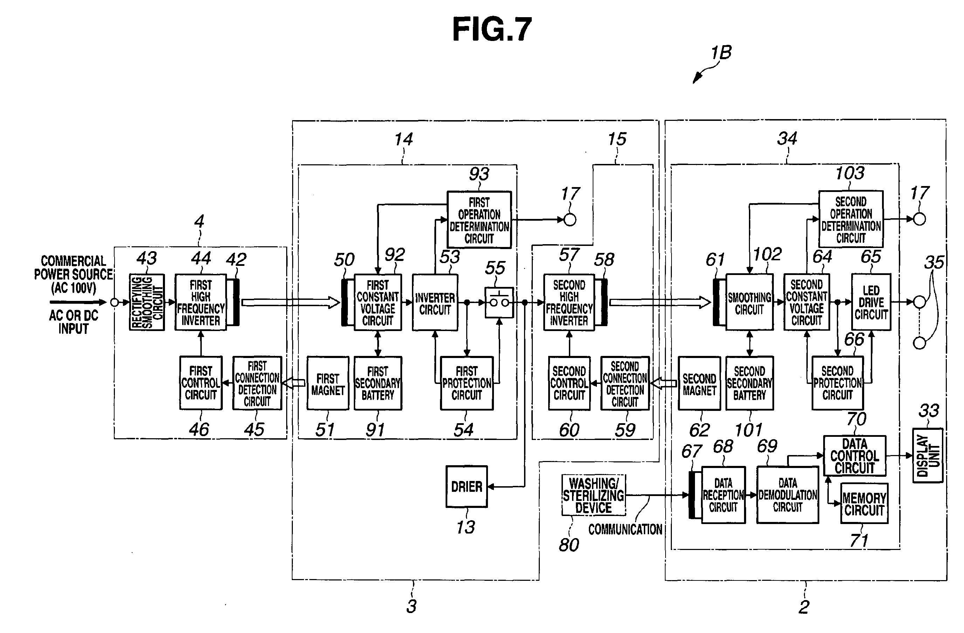

Fig. 5 is an external perspective view showing the entire configuration of a medical instrument storage system (trolley system) of a second embodiment according to the present invention. Fig. 6 is a block diagram primarily showing the electrical structure in the medical instrument storage system (trolley system) shown in Fig. 5. Fig. 7 is a block diagram showing a modified example of the medical instrument storage system (trolley system) shown in Fig. 5.

The basic configuration of the embodiment is substantially the same as that of the first embodiment. The same components as those shown in the first embodiment will be designated with the same reference numerals, and explanations thereof, thus will be omitted. The description with respect to only the difference will be explained hereinafter.

The tray 2 according to the embodiment includes a display unit 33a which displays predetermined information data instead of the display unit 33 of the first embodiment.

The second power receiver 34 is formed as a unit structure which includes a rechargeable secondary battery 101 serving as an electric storage device, and a second operation determination circuit 103 which determines with respect to the operation state of the second power receiver 34 in addition to the configuration as described in the first embodiment.

The secondary battery 101 is a rechargeable electric storage device for storing power supplied from the second power feeder 15 via the second power receiver 34 by the charging circuit (not shown) built in the second power receiver 34. The secondary battery 101 is connected to the smoothing circuit 63 of the second power receiver 34.

Therefore, in the case where appropriate power is supplied to the second power receiver 34, the smoothing circuit 63 serves to smooth the output of the second secondary coil 61 as described above. Meanwhile, when the second power feeder 15 is disconnected from the second power receiver 34 through separation of the tray 2 from the storage case 16 of the trolley unit 3, the second operation determination circuit 103 detects the stopped state of the output, and executes the control for switching the output of the smoothing circuit 63 to that of the secondary battery 101. Accordingly, the second operation determination circuit 103 also serves as the output switching means (unit).

The tray 2 is provided with the operation confirmation LED 17 (see Fig. 6) which is controlled to be lit by the second operation determination circuit 103 when the appropriate power is supplied to the tray 2.

Likewise the first embodiment, the display unit 33a displays the history information under the control of the data control circuit 70. The display unit 33a serves as the display portion for displaying the information (history information) stored in the memory circuit 71 (memory unit). The history information displayed on the display unit 33a includes the results of the processing whether or not the process has been normally performed, the date when the process has been performed, and the ID information of the medical instrument subjected to the processing.

The wireless data communication between the tray 2 and the washing/sterilizing device 80 provides the history information such as the result of washing/sterilizing so as to be displayed on the display unit 33a.

The display unit 33a is arranged on the wall portion of the front surface of the tray 2, having the display screen directed to the front surface. The display unit 33a is exposed such that the display screen of the display unit 33a is confirmed in the state where the tray 2 is set in the storage case 16 of the trolley unit 3.

An inclined portion 2a is formed on the front wall of the tray 2 directed from the upper portion to the front surface such that the display screen of the display unit 33a is confirmed from the front surface in the state where the tray 2 is set in the storage case 16 of the trolley unit 3.

The function of the trolley system 1a thus configured of the present embodiment, which employs the endoscope 8 as the medical instrument will be described referring to Figs. 3 and 5.

Only the function of the embodiment different from that of the first embodiment will be described, and the explanation of the function which has been already described in the first embodiment will be omitted.

Referring to a code B shown in Fig. 3, likewise the first embodiment, the user confirms the ID information of the endoscope 8, washing/sterilizing result, and washing/sterilizing date displayed on the display unit 33a of the tray 2 in execution of the endoscopic inspection or the operation. The user is allowed to select the desired endoscope or the medical instrument among the plural medical instruments including the endoscope stored in the trolley unit 3.

Likewise the first embodiment, the user takes out the tray 2 which stores the washed/sterilized endoscope 8 from the trolley unit 3 in accordance with the inspection to be performed. When the tray 2 is taken out, the distance between the second power feeder 15 and the second power receiver 34 becomes equal to or longer than the predetermined distance, resulting in the disconnection state. The magnetic force of the second magnet 62 is ineffective, which is detected by the second connection detection circuit 59. Upon reception of the detection signal of the second connection detection circuit 59, the second control circuit 60 causes the second high frequency inverter 57 to stop outputting.

The second operation determination circuit 103 detects the stopped state of the output from the second high frequency inverter 57 to execute the control for switching the output of the smoothing circuit 63 to that of the secondary battery 101.

The power from the secondary battery 101 is supplied to the LED drive circuit 65 via the smoothing circuit 63 and the second constant voltage circuit 64 such that the LED drive circuit continuously lights the sterilizing lamp 35. Simultaneously, the power form the secondary battery 101 is supplied to the operation confirmation LED 17 via the second operation determination circuit 103 such that the operation confirmation LED 17 is lit. The power from the second secondary battery 101 is supplied to the display unit 33a and the data control circuit 70 such that the display unit 33a continuously displays the history information of the medical information.

Likewise the first embodiment, the wrapped tray 2 which stores the endoscope 8 is carried to the inspection room. At this time, the secondary battery 101 continuously supplies power to the respective portions of the tray 2.

Referring to a code C shown in Fig. 3, the endoscope 8 which has been used upon the end of the predetermined inspection is stored in the tray 2 so as to be carried to the position where the washing/sterilizing device 80 is installed.

The user sets the tray 2 which stores the used endoscope 8 in the washing vessel of the washing/sterilizing device 80 where a series of the washing/sterilizing process is performed. In the washing/sterilizing process, the stain on the outer surface of the tray 2 may be cleaned by water depending on the level of the hydrophilic property of the photocatalytic portion 37 formed on the outer surface of the tray 2. Accordingly, the tray 2 of the present embodiment improves the detergency performance compared to the tray with no photocatalytic portion 37.

When the washing/sterilizing process is finished, the tray 2 is taken out from the washing/sterilizing device 80 referring to the code A shown in Fig. 3 for performing the wrapping process in the same manner as described above. The secondary battery 101 of the tray 2 continuously supplies power. The clean state inside the tray 2 which stores the endoscope 8 which has been cleaned in the washing/sterilizing process is maintained.

Thereafter the user sets the tray 2 into the storage case 16 of the trolley unit 3. In this way, the washed/sterilized endoscope 8 is stored in the trolley unit 3 in the sanitary state.

As described above, according to the present embodiment, the following advantages may be obtained in addition to the effect of the first embodiment of the present invention, that is, the second power feeder 15 of the tray 2 requires no electric contact due to contactless connection, the power cable does not have to be withdrawn, and the water-proof structure may be easily made. The user is allowed to easily perform the washing/sterilizing of the tray 2 as the single unit entirely.

In the process for washing/sterilizing the medical instrument by setting the tray 2 with the water-proof structure in the washing/sterilizing device 80, the history information of the medical instrument is wirelessly transmitted from the washing/sterilizing device 80 after completion of the washing/sterilizing. Then the data reception circuit 68 of the tray 2 receives the history information via the reception coil 67 or the like such that the data control circuit 70 writes the history information into the memory circuit 71. The history information written into the memory circuit 71 is displayed on the display unit 33a. The user is able to know the type of the medical instrument and the washing/sterilizing result upon the next use of the medical instrument, resulting in easy management.

When the operation determination circuit (hereinafter referred to as the first operation determination circuit in the present embodiment) 56 determines that the power is appropriately supplied from the first power feeder 4, the operation confirmation LED 17 is lit, indicating the appropriate power supply. In contrast, when the power is not appropriately supplied or the power supply is interrupted, the operation confirmation LED 17 is turned off. Thus, the user is able to confirm that the first power feeder 4 is stopped owing to interruption of the power supply as well as the function of the photocatalytic member, and accordingly to take the countermeasure by confirming damage or the connection failure. This makes it possible to easily maintain the system.

Although the second power receiver 34 does not receive the external output, the second operation determination circuit 103 executes the control for switching to the output of the secondary battery 101, thus constantly supplying required power to the tray 2. Therefore, the power supply to the sterilizing lamp 35 of the tray 2 or the display unit 33a is ensured in spite of the carriage of the tray 2 separated from the trolley unit 3. The antibacterial and antifouling functions of the photocatalytic member become effective to constantly maintain the sterilized state and to allow the user to confirm the history information constantly.

As the history information with respect to the medical instrument during the carriage may be constantly displayed on the display unit 33a, such history information as the type of the medical instrument and the date when the washing/sterilizing has been finished may be confirmed any time. Therefore, in the case where the plural trays 2 separated from the trolley unit 3 exist in the inspection room simultaneously, the medical instruments stored in the respective trays 2 may be easily controlled.

As the secondary battery 103 serves to light the operation confirmation LED 17, the user confirms whether or not the power is appropriately supplied to the tray 2 separated from the trolley unit 3.

Next, a modified example of the trolley system of the second embodiment according to the present invention will be described.

Fig. 7 is a block diagram showing the modified example of the trolley system of the second embodiment.

The basic configuration of the modified example is the same as that of the aforementioned embodiment. Accordingly the same components as those of the embodiment are designated with the same codes, and explanations thereof will be omitted. The description with respect to only the difference will be described hereinafter.

A trolley system 1B of the modified example includes a first secondary battery 91 as a rechargeable electric storage device in the first power receiver 14 of the trolley unit 3 in addition to the configuration of the aforementioned embodiment as the different configuration. The secondary battery 101 as the electric storage device in the second power receiver 34 of the tray 2 in the second embodiment will be employed as the second secondary battery 101 in the modified example. The configuration and the function of the second secondary battery 101 are identical to those of the secondary battery 101 in the aforementioned embodiment.

Referring to Fig. 7, the first power receiver 14 in the modified example includes the first constant voltage circuit 92 and the first operation determination circuit 93 serving as both output switching means (unit) and operation determination means (unit) instead of the first constant voltage circuit 52 and the first operation determination circuit 56 in the embodiment.

The first constant voltage circuit 92 of the first power receiver 14 is connected to the rechargeable first secondary battery 91. The first secondary battery 91 is structured to be rechargeable by the charging circuit (not shown) built in the first power receiver 14. The first operation determination circuit 93 determines with respect to the operation state of the first power receiver 14.

In the case where appropriate power is applied to the first power receiver 14, the first constant voltage circuit 92 operates in the same way as the first constant voltage circuit 52 in the aforementioned embodiment. In contrast, in the case where the connection in the first power receiver 14 is interrupted, and the first operation determination circuit 93 detects the stopped state of the output, the control for switching the output of the first constant voltage circuit 92 to that of the first secondary battery 91 is executed. The other operation of the first operation determination circuit 93 is the same as that of the first operation determination circuit 56 in the embodiment as described above.

The second power receiver 34 of the modified example includes a smoothing circuit 102 instead of the smoothing circuit 63 of the aforementioned embodiment, and further includes a second operation determination circuit 103 serving as both the output switching means (unit) and the operation determination means (unit).

The smoothing circuit 102 of the second power receiver 34 is connected to the rechargeable second secondary battery 101. The second secondary battery 101 is formed as the rechargeable electric storage device by a charging circuit (not specifically shown) built in the second power receiver 34. The second operation determination circuit 103 monitors the output of the second constant voltage circuit 64 to determine with respect to the operation state of the second power receiver 34.

In the case where appropriate power is fed to the second power receiver 34, the smoothing circuit 102 operates in the same way as the smoothing circuit 63 of the embodiment. In contrast, in the case where the first power receiver 14 or the second power receiver 34 is disconnected, and the second operation determination circuit 103 detects the stopped state of the output, the control for switching the output of the smoothing circuit 102 to that of the second secondary battery 101 is executed.

The other configuration is substantially the same as that of the embodiment as described above.

In the aforementioned structure, although the connection between the respective power feeder and the power receiver is interrupted, the switching control is executed such that the power is supplied from the first secondary battery 91 and the second secondary battery 101. Accordingly, the inner circuit may be temporarily driven.

Therefore, when the trolley unit 3 is separated from the first power feeder 4 so as to be moved for carrying the endoscope 8, for example, power may be supplied to the drier 13 and the tray 2 of the trolley unit 3.

In other words, when the trolley unit 3 is separated from the first power feeder 4, the first operation determination circuit 93 for monitoring the output of the inverter circuit 53 determines the disconnection state. Then the first operation determination circuit 93 executes the control for switching the output of the first constant voltage circuit 92 to that of the first secondary battery 91. Thus, the power of the first secondary battery 91 is supplied to the drier 13 and the second power feeder 15 of the tray 2 via the first constant voltage circuit 92, the inverter circuit 53, and the output control circuit 55. The second power receiver 34 receives the power from the second power feeder 15 such that the sterilizing lamps 35 are continuously lit.

When the tray 2 is separated from the trolley unit 3, the second operation determination circuit 103 for monitoring the output of the second constant voltage circuit 64 determines the transition to the disconnection state. The second operation determination circuit 103 executes the control for switching the output of the smoothing circuit 102 to that of the second secondary battery 101.

The power from the second secondary battery 101 is supplied to the LED drive circuit 65 via the smoothing circuit 102 and the second constant voltage circuit 64. The LED drive circuit 65 continuously lights the sterilizing lamps 35. The power of the second secondary battery 101 is further supplied to the display unit 33a and the data control circuit 70. The display unit 33a continues to display the history information of the medical instrument.

Thus, whenever the user takes out the tray 2 from the trolley unit 3, the history information as to when the endoscope 8 stored in the separated tray 2 has been washed/sterilized and the like may be confirmed any time. This ensures to execute the history management of the washing/sterilizing process.

The tray 2 is provided with the operation confirmation LED 17. The second operation determination circuit 103 lights the operation confirmation LED 17 in the case where the appropriate power is supplied likewise the first operation determination circuit 93.

In the modified example, the same effects as those of the second embodiment as described above are obtained.

In the modified example, although the first power receiver 14 or the second power receiver 34 does not receive the external output, the first operation determination circuit 93 or the second operation determination circuit 103 execute the control for switching the output to the first secondary battery 91 or the second secondary battery 101. This makes it possible to constantly supply the required power to the trolley unit 3 and the tray 2.

Therefore, when the trolley unit 3 is separated from the first power feeder 4, the predetermined power is constantly supplied to the tray 2 and the drier 13 irrespective of the moving state of the trolley unit 3. Even in the carriage state of the tray 2 separated from the trolley unit 3, the power supplied to the sterilizing lamps 35 of the tray 2, and the display unit 33a may be maintained. The antibacterial and antifouling functions of the photocatalytic member become effective to constantly maintain the sterilized state as well as to constantly confirm the history information.

As the history information with respect to the medical instrument in the carriage state is constantly displayed on the display unit 33a, the history information including the type of the medical instrument, and the date when the washing/sterilizing has been finished can be constantly confirmed. Even if the plural trays 2 having been separated from the trolley units 3 exist in the inspection room simultaneously, the management of the medical instrument stored inside the respective trays 2 may be easily performed.

The second operation determination circuit 103 operates in the same way as the first operation determination circuit 56 of the aforementioned embodiment. Therefore, the user is allowed to confirm whether or not the power is appropriately supplied by the first power feeder 4 and the second power feeder 15 based on the ON/OFF state of the operation confirmation LED 17.

It is to be understood that the present invention is not limited to the embodiments as described above, but may be changed without departing from the scope of the invention.

## Claims

1. A medical instrument storage system comprising:
a power feeder connected to a power source;
a main body which includes a power receiver for receiving a contactless power supply from the power feeder, and a holding portion for holding a medical instrument;
a sanitation keeping unit connected to the power receiver and driven by the power supplied from the power feeder through the power receiver to keep the medical instrument in a sanitary state; and
a moving unit for making the main body movable.

2. The medical instrument storage system according to Claim 1, wherein:
the power feeder is a primary coil connected to the power source; and
the power receiver is a secondary coil electromagnetically coupled with the primary coil.

3. The medical instrument storage system according to Claim 2, wherein the main body includes a detection unit for detecting a state where the power receiver is brought to be close to the power feeder, and a control unit for electromagnetically coupling the primary coil and the secondary coil in accordance with a detection result of the detection unit.

4. The medical instrument storage system according to Claim 1, wherein the power receiver includes a rechargeable secondary battery.

5. The medical instrument storage system according to Claim 1, wherein the sanitation keeping unit is a dehumidifying unit for dehumidifying the medical instrument.

6. The medical instrument storage system according to Claim 1, wherein the sanitation keeping unit is a sterilizing unit for sterilizing the medical instrument.

7. A medical instrument storage system comprising:
a power feeder connected to a power source;
a main body detachably provided with a holding vessel including a holding portion for holding a medical instrument;
an electric storage unit in the holding vessel for storing power supplied from the power feeder; and
a light emitting unit connected to the electric storage unit provided in the holding vessel for emitting a light ray with a predetermined wavelength toward the medical instrument.

8. The medical instrument storage system according to Claim 7, wherein the electric storage unit is installed in a power receiver structured to receive a contactless power supply from the power feeder.

9. The medical instrument storage system according to Claim 8, wherein:
the power feeder is a primary coil connected to the power source; and
the power receiver is a secondary coil electromagnetically coupled with the primary coil.

10. The medical instrument storage system according to Claim 9, wherein the holding vessel further includes a detection unit for detecting a state where the power receiver is brought to be close to the power feeder, and a control unit for electromagnetically coupling the primary coil and the secondary coil in accordance with a detection result of the detection unit.

11. The medical instrument storage system according to Claim 7, wherein the holding vessel includes a reception unit for receiving history information data with respect to the medical instrument wirelessly transmitted from a washing/sterilizing device for washing and sterilizing the medical instrument.

12. The medical instrument storage system according to Claim 11, wherein the holding vessel includes a memory unit for storing the history information data with respect to the medical instrument.

13. The medical instrument storage system according to Claim 12, wherein the holding vessel includes a display unit for displaying the information stored in the memory unit.

14. A medical instrument storage system comprising:
a power feeder connected to a power source;
a main body which includes a power receiver for receiving a contactless power supply from the power feeder, and a holding portion for holding a medical instrument;
a sanitation keeping unit connected to the power receiver and driven by the power supplied from the power feeder through the power receiver to keep the medical instrument in a sanitary state;
a moving unit for making the main body movable;
an electric storage unit provided in the holding vessel for storing power supplied from the power feeder; and
a light emitting unit connected to the electric storage unit provided in the holding vessel for emitting a light ray with a predetermined wavelength toward the medical instrument.
